# EUROPEAN PATENT APPLICATION

(11) **EP 0 995 401 A1**
(43) Date of publication of application: **26.04.2000**
(21) Application number: 99600014.7
(22) Date of filing: 29.09.1999
(51) Int. Cl.: A61B 10/00

(54) **Cervical sampling device with separating head and propeller-like (spiral) axis**

(30) Priority: 19.10.1998 GR 98100385
(71) Applicant: Paganis, Kyriakos G., 19013 Anavyssos, Hellas (GR)
(72) Inventor: Paganis, Kyriakos G., 19013 Anavyssos, Hellas (GR)

(57) **Abstract**

The cervical sampling device with separating head and a central pole with propeller-like wings on its axis comprises a device for the collection of cells consisting of a handle and a head, with or without lengthy fibers on the head as the specialist elects, which with simple movements has the capability to obtain cells from the cervical regions (endo, ecto and the T-zone), which cells lay on the head of the device. After the collection, the head can be easily separated from the handle and placed in a special bottle for its transfer/preservation till it reaches the laboratory.

To the axis of the central pole of the head of the device are affixed flexible propeller-like wings and at its outer end it has a rounded (spherical) tip so as to permit the device to easier enter the cervix and be more effective because the wings on the axis of the central pole, with the appropriate turning motion, open and allow the collection of the cells from the walls of the cervical regions, something required for an effective PAP test.

In the cases of advanced stenosis of the cervix, the device is made only with the handle, the base and the central pole with its propeller-like wings on its axis, as they have been described and are shown on the drawings, so that it permits the specialist the easiest possible entry.

The invention, used to obtain sample cells for medical tests, assures the effectiveness of tests by obtaining a more representative sample from all regions of the cervix. Preserving the head with the cells in an appropriate bottle and using the monolayer method it improves the diagnosis, lessens the costs and minimizes the discomfort of the patient.

## Description

The present invention refers to a device for the collection of cervical cells, consisting of the handle, a head - which can be separated from the handle - and a central pole on the head of the device, which eases the penetration of the device in the cervix, enabling the specialist, with simple movements, to collect fully representative sample cells from the cervix on its head, which is affixed on the handle, and after the collection it is separated from the handle and placed in special bottles destined for the examining laboratory.

### Historical background of the invention

For the selection of cells from the cervical region a number of devices are used today, such as the device known as "spatula", a lengthy "thorny" brush, and a brush with lengthy fibers. The spatula, after it is first placed by the specialist in a way so that its specially formulated end touches the entry of the cervical canal, with circular movements breaks off cells from the external walls of the cervix it comes in contact with and these cells transfer on the surface of the spatula from which they are subsequently collected.

The lengthy "thorny" brush, fixed at the end of a handle, after it is placed by the specialist in the cervical canal, with circular movements of the handle, breaks off cells from its walls, and these cells sit on the bristles of the brush from where they are finally collected.

The brush with the lengthy fibers, set at the end of a handle and consisting of the handle and its head, which is shaped like an arrow and from its base, shaped like a bow, start lengthy fibers equal in length except those in the center which are longer (thus giving the shape of an extended bow), after it is placed by the specialist at the entry of the cervical canal, so as to have the tip of the head inside the canal and its base at the outer walls of the cervix, applying the proper pressure at its handle and with simultaneous circular motions, the tips of the fibers break off cells from the points of contact at the walls of the cervix which cells are held on the fibers of the brush, from where they are collected.

Finally, there is another type of brush the one end of which is shaped as the edge of a spatula while its head at the other end has the shape of a trident (ψ) and its entire head is covered with short equally sized fibers and with appropriate movements by the specialist sample cells are taken from the cervix which cells are placed on the fibers of the head or the surface of the spatula shaped end on the other side of the handle from where the cells are collected.

The collection of cells from the cervical regions using these devices presents major problems. To start with the one piece flat spatula is capable of obtaining sample cells only from the external walls of the cervix; the lengthy thorny brush obtains sample cells almost exclusively from the endo-cervix; the brush with the lengthy fibers limited by its shape but also by the fact that sampling can only be done by the tips of the fibers, is best suited to obtain cells from the external walls of the cervix, less from the T-zone and almost not at all from the endo-cervix; the brush capable of collecting samples on both ends of its handle, appears not to have the ability to collect cells from the T-zone, while it can collect cells from the external walls of the cervix and the endo-cervix.

General characteristic of these devices is the difficulty in obtaining sample cells, simultaneously, from all three regions of the cervix (endo, ecto & T-zone) resulting in inadequate sample cells. However, to have a credible test for which the sample is taken (PAP, etc.) since the diseases (especially cancer) for which the tests are performed and the sample is being taken can be present in one or more regions of the cervix and not others, something which may be due to the age of the patient, it is required that sample cells are obtained from all three regions (the external, T-zone and the internal) of the cervix. This is very hard to do with the present devices, while frequently, to be able to have a complete sample, more pressure is exercised on the handle of the device resulting in topical bleeding frequently causing the sample to be useless and the need for new sampling at a later date.

These disadvantages, which are known to the specialists and have been repeatedly mentioned in congresses and scientific literature (Diagnostic Cytopathology 1992 v. 8 No 5 pg. 492, Acta Cytol-Baltimore-1991 v. 35 pgs. 64-68, J. Reprod Med 1989, v. 34 pg. 629, Diagn Cytopatho) t985 v. 1 pgs. 55-58 Obstet Gynecol 1977 pgs. 25576- 580, Am J Clin Pathol 1974 v. 61 pgs. 285-286, Obstet Gynecol 1959 v. 14 pgs. 133-148) have led specialists to follow the practice of sampling with two different devices on two different occasions. This results in requiring double the time for the screening and the use of the personnel of the laboratory, at twice the cost and twice the discomfort for the patient.

Another equally serious disadvantage in using the devices mentioned to collect sample cells is the required multiple layering movements of their head or collecting end over the slide in order to layer the collected cells upon the slide which is sent to the laboratory for screening under the microscope. Also, using these devices to collect cell samples, over and above the time it requires, has the disadvantage of not collecting samples from a critical region of the cervix (since it is not possible to always obtain sample cells from all cervical regions and place them on the slide) and therefore, the sample that will be sent to the laboratory for screening, may not include cells required by the specialist, resulting in the inability to have a credible diagnosis.

Also, the transfer of the collected cells to the slide, by repeatedly layering the collected cells, results in damaging the collected cells as well as making the screening of the first layers difficult since there are covered by other layers on top. For these reasons, in many cases the specialists require the multiple collection of sample and the creation of multiple slides for screening by the laboratory; but even then it is not assured that there is no loss of critical sample cells, especially when the disease is at its infancy and is not widely spread, in other words, exactly at the time when it can best be treated.

As a matter of fact, it is for these reasons that a new method has been devised for the collection of the sample cells, their maintenance and their submission to technically sophisticated laboratory (monolayer application), with the help of sealed vials which contain preservative liquid. The layering of the cells on the slide is done by using special procedures after processing the sample in vials at the lab, in such a way as to maintain the cells morphologically as well as maintain the entire population of collected cells. The use of the devices used up to now, is not possible with the new method of sample processing since the only way to transfer the collected cells to the preserving liquid is to place in the vial the collecting end of the device and have the specialist hold the device in the liquid while turning it in such a way as to separate the cells from the device, taking care not to spill the liquid and separated cells. However, even if this were done, the inadequacy of the collected sample would continue, as the collecting devices used up to now only collect cells from specific region of the cervix.

### Description of the Device

The cervical sampler with separating head and a central pole with a propeller-like axis, as it is described, has as its objective the effective confrontation of the problems the other devices face in securing a fully representative sample, by the simultaneous collection of sample cells from the three regions of the cervix (ecto, endo and the T-zone), by using only one device, the head of which has a central pole at the axis of which are propeller-like wings with specified dimensions and positioning and which central pole of the head can easily enter the cervical canal of almost all patients, while the entire head can be easily separated from the handle of he device, without any damage to the cells collected or any loss of cells and can then be placed in specially designed bottles for their safe transportation to the laboratory for further processing and diagnosis.

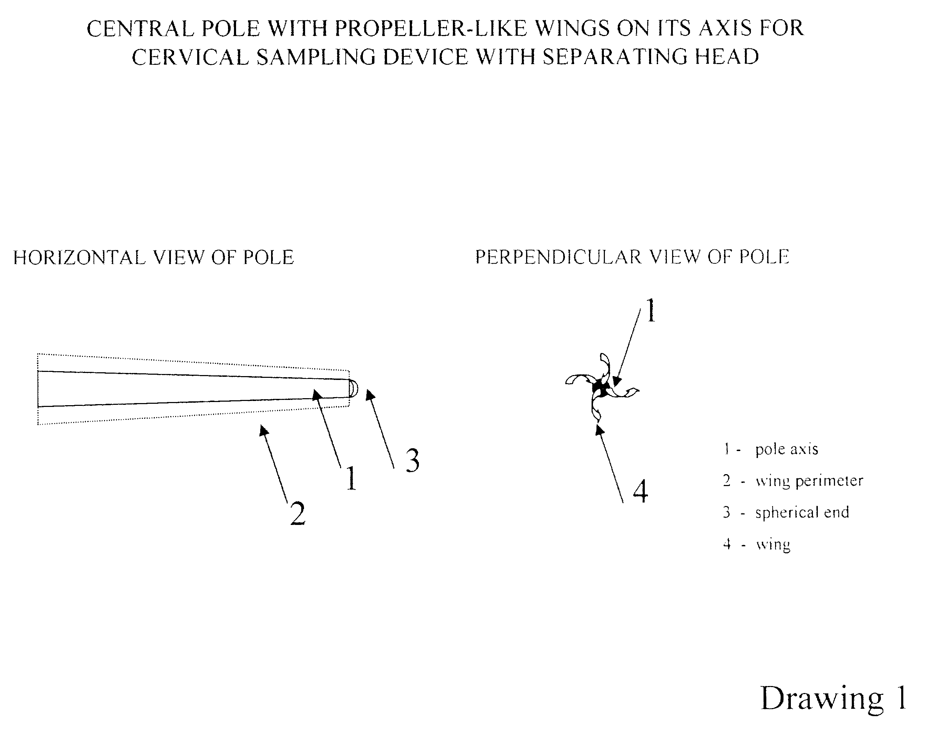

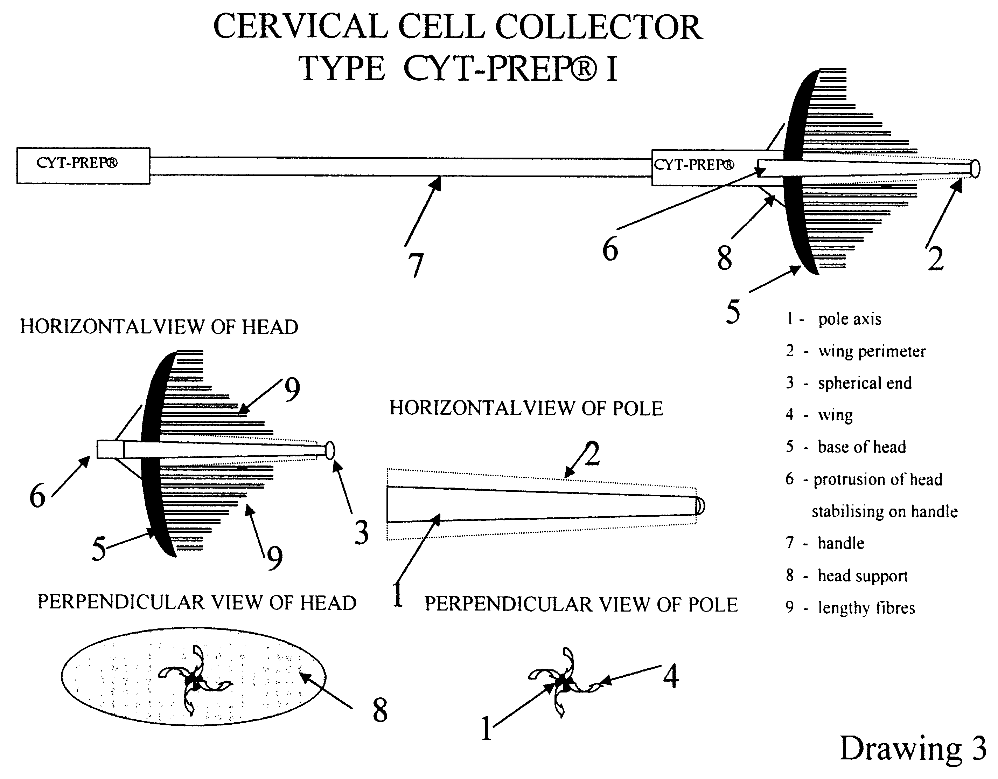

The main advantage of this invention is that the propeller like wings on the axis of the central pole of the head (**Drawing 1**) with their rounded edges allow the entry of the pole of the head in the endo-cervical region and combined with the fibers (**Drawing 3, (9)**) of the head, obtain rich in cells and simultaneously truly representative collection of cells; separating the head from the handle, it is then placed in special bottle, assuring that no damage is done to the morphology of the cells up to the processing of the sample at the lab and the examination of the collected cells so that the diagnosis is more secure, the tests cheaper and the discomfort to the patient lesser.

**Drawing 1** represents the horizontal and vertical sections of the central pole of the head. On the horizontal section the continuous inside line of the drawing represents the central axis (**1**) of the pole while the dotted line represents the external perimeter of the wings (**2**). The vertical section represents the central axis (**1**) at the center of the drawing and the wings (**4**), which continue, increasing in size, for the entire length of the axis. The spherical external edge (**3**) allows the easy entry of the central pole of the head in the endo-cervical region.

The central pole of the head (**Drawing 1**) of the device is characterized by the fact that it is cone shaped, the length from the base is 2.2 CMS and the wings have the shape of a propeller wing (helictical). The total width of the central pole at the base, including the wings, is 3,6 mm, while at he external end of the pole there is a spherical end (**3**) the radius of which is 0,74 mm. The axis of the pole (**1**) is square but with rounded sides and starts at the base with a radius of 0,5 mm, while at the upper end the radius is reduced to 0,3 mm. The four (4) wings (**4**) are found at the four corners of the axis and the distance of the end of each wing from the base of the previous wing is 1,5 mm. The pole, at its tip, the point of entry, has rounded shape (**3**) so as to have a smooth surface. At the length of the pole, there are flexible propeller-like wings (**4**). The thickness of the wings is 0,3 mm, while the radius of the wings is 0,15 mm. The propeller like wings bend in the same direction; in other words they are positioned as if they were two, crossed in the center, letters "**S**".

The propeller like wings, are constructed so that the entry in the cervical canal can be easier and this is because during the entry of the device into the cervical canal the specialist turns the device toward one direction, so as to ease the entry, because the wings bend (close) easily in that direction and wrap around the central axis of the pole. However, when the head is at the right position, the specialist reverses the direction and turning the device the opposite direction, allows the wings to open and in this manner they are in a position to collect valuable cells by "scraping" the walls of the endo-cervix and the T-zone. When the sample is taken, the specialist, can again reverse the direction, so as to ease the exit of the device painlessly. The distance between the wings as well as their length is such as not to allow one to cover the other reducing the volume of the portion of the pole in the endo-cervix. Also, when the turning is reversed to collect cells, the wings do not overlap and in this manner one does not affect the functioning of the other in the collection process.

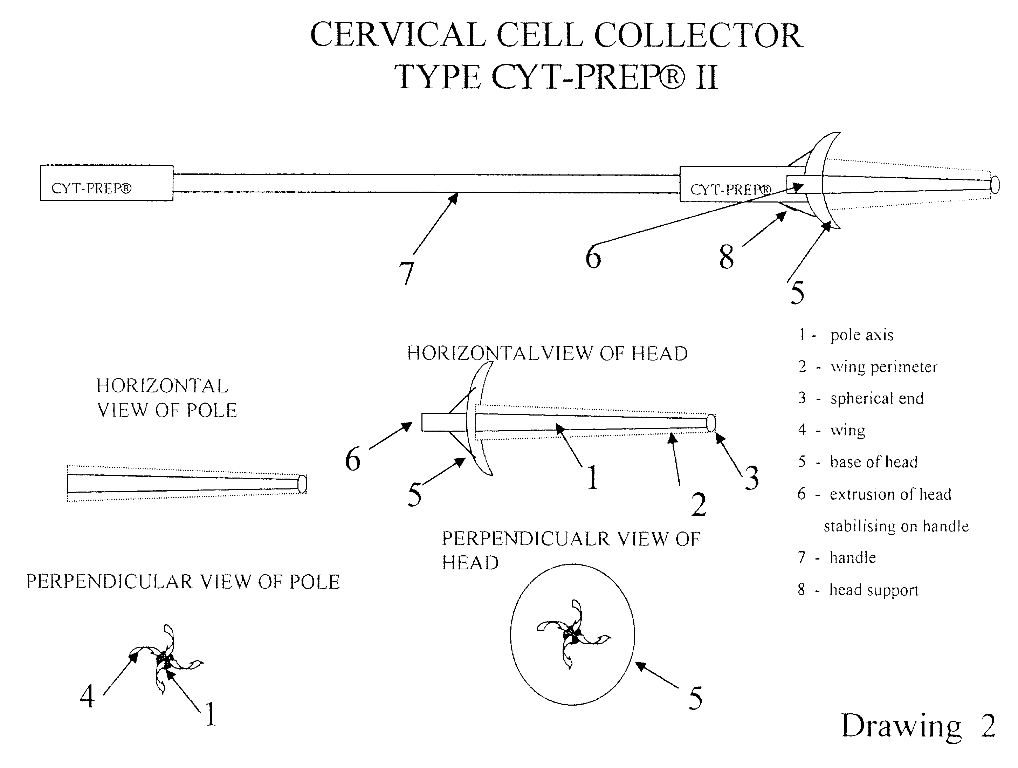

Because the cervix of each patient differs as to the stenosis of the T-zone of each patient, the central pole is placed on two types of heads for cervical sampling devices (**Drawings 2 & 3**) in such a way so as to allow the specialist to select the type of head which will permit him the easier penetration of the cervical canal in conjunction with its condition and the required sampling.

**Drawing 2** depicts one type of device in its entirety that will be used in cases of extreme stenosis, the CYT-PREP® **II**. Its total length of the cervical sampling device CYT-PREP® **II** is approximately 24 CMS.

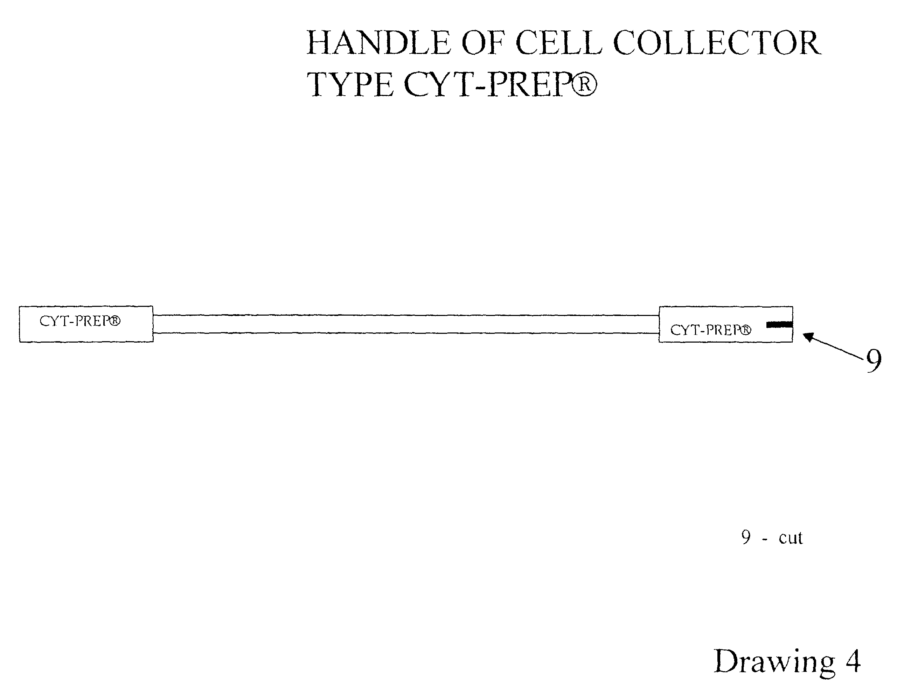

The handle (**7**) is made of tough plastic, which can bend slightly. Its vertical section shows a six-angle shape, which allows the easy and accurate placing of the device by the specialist using the fingers of one hand, and its length is approximately 21 CMS. The width of the handle is 0.35 CM, except for its ends, which are thicker reaching approximately 0,5 cm. At the upper end of the handle there are two small indentations (**Drawing 4**, (**9**)) which permit the penetration of the head supports (**8**) of the base of the head; also there is a cavity reaching approximately 1 CM inside the handle, shaping in this way the reception area in which the protrusion of the head (**6**) is placed, so that the two pieces (head & handle), making up the device, are absolutely stable.

**Drawing 2** also depicts the head of device CYT-PREP®**II**, which is used by the specialist in the case of stenosis of the cervical canal. The base of the head (**5**) has the shape of a satellite dish and its diameter is approximately 1 CM.

At the bottom end of the base and at its center, perpendicular to its main body there is a stabilizing protrusion (**6**) approx. 1 CM long, which is inserted to the upper end of the handle. To the right and left of the protrusion there are two triangular supports (**8**) which reach to the bow of the head, which supports are inserted in the cuts (**Drawing 4**, (**9**)) on the top of the handle securing the device (its head to its handle) during use. A sectional look gives the impression of a circle. From the middle of the internal bow of the base of the head, starts a lengthy pole with a central axis, approximately 2.20 CMS in length. The pole, at it outer edge has a spherical shape so that the surface is smooth. During its entire length, there are propeller-like flexible wings (**Drawing 1**).

The central pole is as described above (**Drawing 1**). The order of the wings, as described, give the opportunity to the user of the device, with appropriate movements and without exercising any pressure, to obtain the cells from the endo-cervical region of the patient, even when she is suffering from stenosis of the T-zone, collecting cells at the tips of the wings.

Thereafter, the specialist can, with a small flip of one finger at the bottom of the head, separate it from the handle and place it in a special bottle, which is sealed, with the head inside, and sent to the laboratory for processing, using the monolayer application method.

**Drawing 3** depicts the device **CYT-PREP® I**, which is used by the specialist in most cases and which has the capability to obtain sample cells from all the regions of the cervix (endo, ecto & T-zone). The base of the head (**Drawing 3, (5)**) has the shape of a torch, the conceivable cord of which has a length of approximately 2,20 CMS.

At the bottom end of the base and at its center, perpendicular to its main body there is a stabilizing protrusion (**6**) approx. 1 CM long, which is inserted to the upper end of the handle. To the right and left of the protrusion there are two triangular supports (**8**) which reach to the bow of the head, which supports are inserted in the cuts (**Drawing 4, (9)**) on the top of the handle securing the device (its head to its handle) during use. From the middle of the internal bow of the base of the head, starts a lengthy pole with a central axis, approximately 2.20 CMS in length. The pole, at it outer edge has a spherical shape so that the surface is smooth. During its entire length, there are propeller-like flexible wings (**Drawing 1**).

The sectional view of the head gives a picture of a parallelogram with rounded corners. From the upper part of the base of the head (**5**), in other words inside the bow, flexible fibers start perpendicular to the base (**9**), the length of which increases progressively toward the center of the base, where the length of the longer fibers reaches 1.50 CMS.

From the inside center of the bow of the base of the head starts a lengthy extrusion, central pole, with a length of 2.20 CMS. At the point of penetration, the tip is rounded so that its surface is smooth. At the length of the central axis of the pole, exist flexible propeller-like wings (**Drawing 1**).

The central pole is as described above (**Drawing 1**). The order of the wings, as describe, gives the opportunity to the user of he device, with appropriate movements and without exercising any pressure, to obtain the cells from the endo-cervical region of the patient as well as from the T-zone and the ecto-cervical region, which are collected at the tips of the wings and upon the lengthy fibers.

Thereafter, the specialist can, with a small flip of one finger at the bottom of the head, separate it from the handle and place it in a special bottle, which is sealed, with the head inside, and sent to the laboratory for processing, using the monolayer application method.

## Claims

1. Cervical sampling device with separating head, from its handle (**7**), characterized by the fact that at the base of its head (**5**) with or without lengthy fibers (**9**), there is a central pole with an axis (**1**) which carries propeller-like wings (**4**) from flexible material, the size of which continues to increase upon the entire length of the axis (**1**) and have same direction so as to open or close simultaneously with the appropriate turn of the device.

2. Cervical sampling device with separating head , from the handle (**7**), in accordance with claim 1, is characterized by its central pole on its head, the axis of which is conical, its length from the base of its head (**5**) is approximately 2,2 CM while its width starts at 1.48 MM at the upper end (**2**) and reaches 3.5MM at the base of the head, with the wings (**4**) in the position as they are produced.

3. Cervical sampling device with separating head, from the handle (**7**), in accordance with claim 1 and 2, is characterized from the fact that its central pole on its head (**5**), with or without lengthy fibers, with its propeller-like wings on its axis (**1**), at its outer tip has a rounded (sphere-like) shape (**3**) with a radius of 0.74 mm, appropriate for the easy penetration of the cervical canal.

4. Cervical sampling device with separating head, from the handle (**7**), in accordance with claim 1, characterized by the fact that its axis with the propeller-like wings (**1**) of the central pole has a square shape with rounded corners which start with a radius of 0,5 mm and end at he outer edge with a radius of 0,3 mm.

5. Cervical sampling device with separating head, from the handle (**7**), in accordance with claim 1 and 4, characterized by the fact that the propeller-like wings (**4**) are found at the rounded corner of the quadrangular axis of the central pole (**1**) and have a length of 0.3 mm and are flexible and uniform.

6. Cervical sampling device with separating head, from the handle (**7**), in accordance with claim 1 and 5, characterized by the fact that its propeller-like wings (**4**) of the central pole (**1**) of the head (**5**) with or without lengthy fibers (**9**), end in rounded ends with a surface which has a radius of approximately 0,15 mm appropriate for the easy entry of the sampling device in the cervical canal with a turning motion toward one direction, while when reversing the direction, collect a rich in cells sample from the cervical regions.

7. Cervical sampling device with separating head, from the handle (**7**), in accordance with claim 1 and 6, is characterized by the fact that from each wing of the axis (**1**) of the central pole of the head (**5**) with or without lengthy fibers (**9**), has equal distance from the preceding, in such a way so that when the wings fold, one does not cover the other and create a larger volume or when they unfold with the turning of the handle, one would not cover the other hindering the collection of the cells.

8. Cervical sampling device with separating head, from the handle (**7**), in accordance with claim 1, characterized by the fact that the separating head without the lengthy fibers (**Drawing 2**) on the base (**5**) of which is affixed the central pole with the propeller-like wings on its axis, is selected by the specialist for the simultaneous collection of samples cells from the endo-cervical and T-zone regions of the cervix, if the patient is suffering from extreme stenosis.

9. Cervical sampling device with separating head, from the handle (**7**), in accordance with claim 1, characterized by the fact that the central pole of the head with the lengthy fibers, is approximately 1 CM longer than the longer fibers, so as to have only the central pole make an easier entry in the cervical canal to obtain cells.

10. Cervical sampling device with separating head, from the handle (**7**), in accordance with claim 1 & 7, characterized by the fact depending upon the direction the head is turned, the width of the central axis with the wings affixed varies from +40% to -40% from that when the device is not being used, for the easier entry and better cell collecting ability.
